# EUROPEAN PATENT APPLICATION

(11) **EP 4 400 124 A1**
(43) Date of publication of application: **17.07.2024**
(21) Application number: 23305052.5
(22) Date of filing: 13.01.2023
(51) Int. Cl.: A61L 27/38, A61L 27/46, A61L 27/54, B33Y 70/10, B33Y 80/00, B33Y 10/00

(54) **FORMULATION FOR 3D PRINTING BONE IMPLANTS AND METHODS FOR PREPARING THE SAME**

(71) Applicant: Nantes Université, 44000 Nantes (FR); CHU de Nantes, 44000 Nantes (FR); Institut National de la Santé et de la Recherche Médicale (INSERM), 75013 Paris (FR)
(72) Inventor: Weiss, Pierre, 44340 Bouguenais (FR); Charbonnier, Baptiste, 44000 Nantes (FR); Léna, Guyon, 44440 Joué-sur-Erdre (FR)
(74) Representative: Cabinet Nony

(57) **Abstract**

The present invention relates to the field of personalized medicine.

In particular, the invention relates to a formulation comprising (**i**) a calcium phosphate cement or a magnesium phosphate cement and (**ii**) an hydrogel of polysaccharides, for 3D printing bone implants; characterized in that the polysaccharides comprise at least one selected from: silylated hyaluronic acid and silylated chitosan.

## Description

### TECHNICAL FIELD

The present invention relates to the field of personalized medicine.

The present invention also relates to the field of 3D printing, and particularly the 3D printing of bone implants which are suitable for bone regeneration and bone repair.

The present invention also relates to formulations for preparing such bone implants, and corresponding kits.

The present invention also relates to formulations which are suitable for storage in the long-term, and with improved mechanical properties.

The present invention further relates to methods for preparing such formulations, as well as methods for 3D printing bone implants.

### BACKGROUND

The bone is a complex organ endowed with innate regenerative capacities. Therapeutic strategies for bone repair and/or bone regeneration typically include the use of synthetic biomaterials which may replace autologous bone graft.

Synthetic biomaterials for bone regeneration have been reported in the Art. Ceramics have widespread clinical use, due to their biocompatibility and osteoconduction properties. However their use for, specifically, bone repair is hindered by their low bioactivity, low biodegradation and fragility.

There is thus a general need for biomaterials which can be stored in a sterile manner over long term, while remaining biocompatible and biodegradable.

Cements have also been reported as synthetic materials in minimally invasive applications, due to their improved biodegradation properties over ceramics, while being compatible with the addition of other biological compounds. Yet, cements also tend to have lower mechanical properties and no interconnected macroporosity.

Recent developments have allowed for the custom production of calcium phosphate (CaP) scaffolds through additive manufacturing, which allows for the personalized treatment of patients. However, 3D printed calcium phosphate bone implants are often brittle, should be handle with utmost care by the physician, and have to be placed without breaking in complex bone defect which remains very challenging today.

In addition, due to the inaccuracies induced by the clinical (e.g., CT-scan) and production workflow (e.g., additive manufacturing), mismatch between the produced personalized implant and the defect to fill is common, which may significantly hinder bone repair.

WO 2021/209616 teaches a cement formulation for bone repair, which is characterized by the association of a phosphocalcic cement (CPC) and of an hydrogel of polysaccharides. The proposed cement formulation is found to be compatible with 3D printing.

There is still a need for biomaterials mimicking the mechanical characteristics of the bone. This is particularly the case for applications directed toward personalized medicine, with require 3D bone implants to be manipulated and inserted into complex injuries or bone defects, and further maintain contact of the implanted biomaterial with bone wall of the cavity.

Such 3D bone implants generally require not only to be hardened and rigidified *in situ,* but also to remain sufficiently elastic to be deformed and/or for the pratician to insert them beforehand, for example after 3D printing or before surgery, in order to adapt them to the corresponding patient.

Such a situation may occur, for example, for the treatment of cleft lip/palate defects, which consist of a congenital deformity requiring a multi-step treatment, including an early soft tissue repair and then a hard tissue repair of the hard palate and the alveolar cleft.

There is thus a need for methods and biomaterials which are applicable to the treatment of complex bone injuries, and/or for improving bone regeneration and bone repair.

There is thus a need for methods and biomaterials with novel biomechanical properties, which remain compatible with 3D printing.

There is thus a need for bone implants, especially 3D bone implants, with improved elastic properties, namely high deformation properties without breaking and restoration of size after strength, which also remain resistant *in situ,* and compatible with therapeutic use.

There is thus a need for 3D bone implants that can be handled and adapted on the fly (e.g., cut with scalpel, or deformed without breaking) in order to ensure a perfect contact between the scaffold and the edges of the bone defect.

There is also a need for bone implants, especially 3D bone implants, with osteoinductive properties.

The invention has for purpose to meet the above-mentioned needs.

### SUMMARY

According to a **first main embodiment,** the invention relates to the use of a formulation comprising **(i)** a calcium phosphate cement or a magnesium phosphate cement and **(ii)** an hydrogel of polysaccharides, for 3D printing bone implants; characterized in that the polysaccharides comprise at least one selected from: silylated hyaluronic acid and silylated chitosan.

According to a **second main embodiment,** the invention relates to a kit for 3D printing of bone implants comprising:
(i) a calcium phosphate cement and/or a magnesium phosphate cement; and
(ii) silylated non-reticulated polysaccharides, said polysaccharides comprising at least one selected from: silylated hyaluronic acid and silylated chitosan polysaccharides.

According to a **third main embodiment,** the invention relates to a method to prepare a formulation for 3D printing bone implants, comprising a step of bringing into contact (i) a calcium phosphate cement or a magnesium phosphate cement and (ii) silylated non-reticulated polysaccharides comprising at least one selected from : silylated hyaluronic acid and/or silylated chitosan polysaccharides.

According to a **fourth main embodiment,** the invention relates to a method to prepare a 3D bone implant, comprising at least the steps of :
a) providing a formulation comprising **(i)** a calcium phosphate cement or a magnesium phosphate cement and **(ii)** an hydrogel of polysaccharides comprising at least one selected from: silylated hyaluronic acid and silylated chitosan;
b) 3D printing said formulation, thereby producing the bone implant;
c) bringing the bone implant in contact with a medium, such as a biological medium and/or a medium with pH equal or superior to 4.

According to a **fifth main embodiment,** the invention relates to a method to prepare a 3D bone implant, comprising at least the steps of :
a) providing a formulation comprising **(i)** a calcium phosphate cement and **(ii)** an hydrogel of polysaccharides;
b) 3D printing said formulation, thereby producing the bone implant;
c) bringing the bone implant in contact with a medium, such as a biological medium and/or a medium with pH equal or superior to 4;
d) further modifying the bone implant after step d), thereby producing a customized form of the bone implant.

### DESCRIPTION OF THE FIGUREES

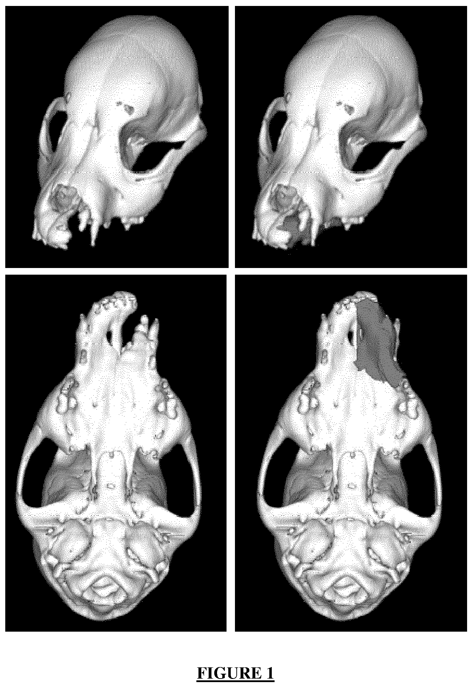

**Figure 1****. Computed Tomography 3D Scanner of an american cocker affected by cleft palate deformity.**

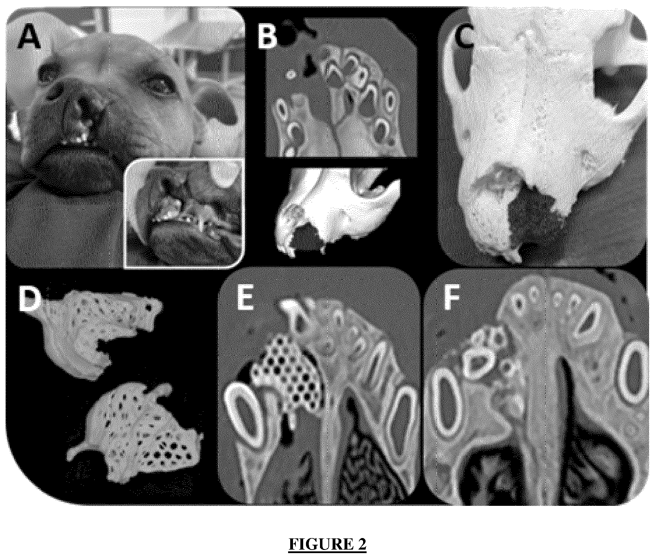

**Figure 2****. CT scan three months after surgery, including the osteointegrated scaffold. Biodegration of the scaffold and bone formation are observed *in situ.* 2A:** picture of a dog having an unilateral cleft lip and palate. **2B** and **2C**, preoperative CT scan 2 weeks prior to surgery and design of a custom implant and simulation of surgery using 3D polymer models. **Figure 2D****,** custom implant designed in two parts adapted to facilitate insertion into the defect. **Figure 2E****,** CT scan after reconstructive surgery. **Figure 2F****,** CT scan at 3 months after surgery showing bone formation capable of supporting eruption of permanent teeth.

### DETAILED DESCRIPTION

The inventors propose herein novel formulations for the preparation of cements, combining **(i)** a calcium phosphate cement or a magnesium phosphate cement and **(ii)** a hydrogel comprising a selection of silylated polysaccharides, which are compatible with the 3D printing of bone implants. The selection of silylated polysaccharides according to the present disclosure comprises at least one selected from: silylated hyaluronic acid and silylated chitosan polysaccharides. Those formulations are also reported hereafter as *"hybrid"* cements.

In particular, it has been found that the bone implants which can be produced with those formulations, after 3D printing, possess advantageous transitory elastic properties following hydration, as illustrated experimentally with three-point flexural tests.

The 3D bone implants are also found to be compatible with long-term storage, after a drying or a sterilization step.

Also, surprisingly, those novel mechanical properties are compatible with the general constraints expected for a bone implant, in particular their ability to become and stay rigid *in situ,* while also being compatible with bone regeneration.

Surprisingly, those properties are shared for, both, calcium phosphate cements (e.g., apatitic cements or brushitic cements) and magnesium phosphate cements, in combination with an hydrogel of silylated polysaccharides.

According to a **first main embodiment,** the invention relates to the use of a formulation comprising **(i)** a calcium phosphate cement or a magnesium phosphate cement and **(ii)** an hydrogel of polysaccharides, for 3D printing bone implants; characterized in that the polysaccharides comprise at least one selected from: silylated hyaluronic acid and silylated chitosan.

According to a particular embodiment, the hydrogel in the formulation is a hydrogel of silylated hyaluronic acid.

According to a particular embodiment, the hydrogel in the formulation is a hydrogel of silylated chitosan.

According to a particular embodiment, the hydrogel in the formulation further comprises one or more additives, such as those selected from the group consisting of: gelatin, carboxymethyl cellulose, hydroxypropyl methylcellulose, methylcellulose, hydroxyethyl cellulose, other cellulose derivatives, alginate, hyaluronic acid, collagen, sodium salts, polyvinyl pyrrolidones, polyvinyl alcohol, arabic gum, guar gum, xantham gum, chitosans, poloxamers or a combination thereof.

Thus, according to a particular embodiment, the hydrogel in the formulation further comprises one or more additives selected from the group consisting of: hydroxypropylmethylcellulose (HPMC), hydroxyethylcellulose (HEC), carboxymethylcellulose (CMC), or a functionalized (e.g., silylated) form thereof, native hyaluronic acid, starch, and poloxamers (e.g., pluronic F127), and combinations thereof.

According to a particular embodiment, the hydrogel is a hydrogel of polysacharides further comprising hydroxypropylmethylcellulose (HPMC) or a silylated form thereof.

According to a particular embodiment, the hydrogel is thus a hydrogel of polysacharides comprising at least one selected from: silylated hyaluronic acid and silylated chitosan; and further comprising hydroxypropylmethylcellulose (HPMC) or a silylated form thereof

According to a particular embodiment, the hydrogel is thus a hydrogel of polysacharides comprising at least one selected from: silylated hyaluronic acid; and further comprising hydroxypropylmethylcellulose (HPMC) or a silylated form thereof

According to a particular embodiment, the hydrogel is thus a hydrogel of polysacharides comprising at least one selected from: silylated chitosan; and further comprising hydroxypropylmethylcellulose (HPMC) or a silylated form thereof.

According to a particular embodiment the cement is a calcium phosphate cement; for example an apatitic cement or a brushitic cement; and in particular an apatitic cement. According to a more particular embodiment, said calcium phosphate cement has alpha tricalcium phosphate (α-TCP) as a precursor.

According to another particular embodiment, the cement is a magnesium phosphate cement.

According to a particular embodiment, the cement is a phosphocalcic cement (especially a calcium phosphate cement as defined above), and the hydrogel is a hydrogel of polysacharides comprising at least one selected from silylated hyaluronic acid and silylated chitosan ; further comprising hydroxypropylmethylcellulose (HPMC) or a silylated form thereof.

According to a particular embodiment, the cement is a magnesium phosphate cement, and the hydrogel is a hydrogel of polysacharides comprising at least one selected from silylated hyaluronic acid and silylated chitosan ; further comprising hydroxypropylmethylcellulose (HPMC) or a silylated form thereof.

According to a particular embodiment, the mass ratio hydrogel/cement is of 2 :3 w/w.

According to a particular embodiment, said formulation comprises from 1 to 30% w/w of silylated polysaccharide, compared to the total weight of the formulation ; which may thus include 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30% w/w of silylated polysaccharide, compared to the total weight of the formulation.

According to a particular embodiment, said hydrogel comprises from 1 to 30% w/w of silylated polysaccharide, compared to the total weight of the hydrogel ; which may thus include 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30% w/w of silylated polysaccharide, compared to the total weight of the hydrogel.

According to a particular embodiment, said hydrogel comprises from 1 to 10% w/w of silylated hyaluronic acid and/or silylated chitosan, compared to the total weight of the hydrogel.

According to examplified embodiment, said hydrogel comprises from 2 to 8% w/w of silylated hyaluronic acid and/or silylated chitosan, compared to the total weight of the hydrogel.

According to examplified embodiment, said hydrogel comprises from 1 to 4% w/w of hydroxypropylmethylcellulose, compared to the total weight of the hydrogel.

According to a **second main embodiment,** the invention relates to a kit for 3D printing of bone implants comprising :
(i) a calcium phosphate cement and/or a magnesium phosphate cement; and
(ii) silylated non-reticulated polysaccharides, said polysaccharides comprising at least one selected from: silylated hyaluronic acid and silylated chitosan polysaccharides.

The silylated non-reticulated polysaccharides which are part of the kits according to the present disclosure, and/or which are brought into contact with the calcium phosphate cement or magnesium phosphate cement of the invention may thus be present in the form :
- of non-reticulated silylated hyaluronic acid and/or non-reticulated silylated chitosan polysaccharides, which are not part of a physical hydrogel ; and/or
- of a covalent hydrogel.

Advantageously, when the cement and the silylated non-reticulated polysaccharides are brought into contact, an hydrogel of said polysaccharides is formed, thereby contributing to the advantageous properties of the formulation.

According to a **third main embodiment,** the invention relates to a method to prepare a formulation for 3D printing bone implants, comprising a step of bringing into contact (i) a calcium phosphate cement or a magnesium phosphate cement and (ii) silylated non-reticulated polysaccharides comprising at least one selected from : silylated hyaluronic acid and/or silylated chitosan polysaccharides.

According to a **fourth main embodiment,** the invention relates to a method to prepare a 3D bone implant, comprising at least the steps of :
a) providing a formulation comprising **(i)** a calcium phosphate cement or a magnesium phosphate cement and **(ii)** an hydrogel of polysaccharides comprising at least one selected from: silylated hyaluronic acid and silylated chitosan;
b) 3D printing said formulation, thereby producing the bone implant, and optionally drying the bone implant;
c) bringing the bone implant in contact with a medium, such as a biological medium and/or a medium with pH equal or superior to 4.

According to one particular embodiment, the invention relates to a method to prepare a 3D bone implant, comprising at least the steps of :
a) bringing into contact (i) a calcium phosphate cement or a magnesium phosphate cement and (ii) silylated non-reticulated polysaccharides comprising at least one selected from : silylated hyaluronic acid and/or silylated chitosan polysaccharides; thereby providing a formulation for 3D printing comprising an hydrogel of said polysaccharides;
b) 3D printing said formulation, thereby producing the bone implant, and optionally drying the bone implant;
c) bringing the bone implant in contact with a medium, such as a biological medium and/or a medium with pH equal or superior to 4.

According to a **fifth main embodiment,** the invention relates to a method to prepare a 3D bone implant, comprising at least the steps of :
a) providing a formulation comprising **(i)** a calcium phosphate cement and **(ii)** an hydrogel of silylated polysaccharides;
b) 3D printing said formulation, thereby producing the bone implant, and optionally drying the bone implant;
c) bringing the bone implant in contact with a medium, such as a biological medium and/or a medium with pH equal or superior to 4;
d) further modifying the bone implant after step d), thereby producing a customized form of the bone implant.

According to one particular embodiment, the invention relates to a method to prepare a 3D bone implant, comprising at least the steps of :
a) bringing into contact (i) a calcium phosphate cement or a magnesium phosphate cement and (ii) silylated non-reticulated polysaccharides comprising at least one selected from : silylated hyaluronic acid and/or silylated chitosan polysaccharides; thereby providing a formulation for 3D printing comprising an hydrogel of said polysaccharides;
b) 3D printing said formulation, thereby producing the bone implant, and optionally drying the bone implant;
c) bringing the bone implant in contact with a medium, such as a biological medium and/or a medium with pH equal or superior to 4;
d) further modifying the bone implant after step d), thereby producing a customized form of the bone implant.

Advantageously, it is found herein that the 3D printed formulations according to the invention are endowed with advantageous transitory elastic properties when brought in contact with said medium, and in particular said biological medium. This step is further disclosed herein as a « *hydration »* step. Alternatively, this step may also be reported herein as a « *re-hydration step* » when the method includes a plurality of « *hydration* » steps.

For example methods for preparing the 3D bone implant may comprise or or more additional « drying » steps, before or after the said « hydration » or « rehydration » steps.

According to some particular embodiments, the methods for preparing the 3D bone implant may further comprise one or more steps of storage (e.g., at least for days or months) after the drying step(s).

According to some particular embodiments, the methods for preparing the 3D bone implant may comprise one or more steps of storage (e.g., at least for days or months) after c) and before step d).

The time of hydration or re-hydration may vary, for example in minutes, hours or days. In a non-exhaustive manner, the step of bringing the bone implant in contact with the medium may thus be of at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 160, 170, 180, 190, 200, 300, 400, 500, 600, 700, 800, 900, 1000 minutes or more than 1000 minutes.

Those methods for preparing 3D bone implants are particularly convenient as *in vitro* or *ex vivo* methods.

Examples of mediums which may be considered in the above-mentioned methods include any medium/composition/solution, for example any medium with a pH equal or superior to 1, 2 or 3 ; in particular with a pH equal or superior to 4, and preferably with a pH equal or inferior to 10.

Accordingly, examples of such mediums may comprise or consist of a NaH₂PO₄ solution, a phosphate saline buffer (PBS) solution, or any other biocompatible buffer, such as any buffer or otherwise pharmaceutically acceptable carrier.

According to some particular embodiments, the medium may comprise or consist of a biological composition.

According to some particular embodiments, the medium may comprise or consist of a blood sample or a blood-derived sample, or a bone marrow sample, stem cells, and compositions thereof.

According to a particular embodiment, the methods to prepare a 3D bone implant, according to the present invention may further include one or more additional steps of modifying the bone implant, thereby producing a customized form of the bone implant.

According to some embodiments, the one or more additional steps of modifying the bone implant may be achieved after the hydration step (i.e. the step of bringing the bone implant in contact with a medium).

According to some embodiments, the one or more additional steps of modifying the bone implant may be achieved before the hydration step (i.e. the step of bringing the bone implant in contact with a medium).

According to some embodiments, the one or more additional steps of modifying the bone implant may be achieved before and after the hydration step (i.e. the step of bringing the bone implant in contact with a medium).

According to some embodiments, the one or more additional steps of modifying the bone implant may be achieved before or after a sterilization step.

A sterilization step may be performed, for example, by a short cycle of steam sterilization, more particularly bowie dick can be used (for example at 134°C during at least 3 minutes).

According to a particular embodiment, the above-mentioned methods may further comprise a step of bringing into contact (e.g., mixing) the cement and/or the polysaccharides, for example the silylated polysaccharides, or hydrogel thereof, with a biological material prior to 3D printing.

According to a particular embodiment, the above-mentioned methods may further comprise a step of bringing into contact (e.g., mixing) the cement and/or the polysaccharides, for example the silylated polysaccharides, or hydrogel thereof, with a biological material after 3D printing.

According to particular embodiments, the above-mentioned methods may comprise a step of bringing into contact the phosphocalcic cement or the magnesium phosphate cement with a biological material, thereby preparing a first precursor formulation, and then bringing into contact the first precursor formulation with the silylated polysaccharides, or hydrogel thereof.

According to other particular embodiments, the above-mentioned methods may comprise a step of bringing into contact the silylated polysaccharides, or hydrogel thereof, with a biological material, thereby preparing a first precursor formulation, and then bringing into contact the first precursor formulation with the phosphocalcic cement or the magnesium phosphate cement.

According to other particular embodiments, the above-mentioned methods may comprise a step of bringing into contact the formulation and the biological material before 3D printing the formulation, or during the step of 3D printing.

According to other particular embodiments, the above-mentioned methods may comprise a step of bringing into contact the bone implant and the biological material, thus after, immediately after or during 3D printing.

According to a **sixth main embodiment,** the invention relates to an implant prepared with a formulation comprising **(i)** a calcium phosphate cement or a magnesium phosphate cement and **(ii)** an hydrogel of polysaccharides, for 3D printing bone implants; characterized in that the polysaccharides comprise at least one selected from: silylated hyaluronic acid and silylated chitosan

According to a particular embodiment, the implant and/or the formulation may further comprise a biological material, and in particular growth factors and/or cells.

According to a particular embodiment, the cement used in the formulation according to the invention is in powder form, more particularly in the form of grains with an average size of less than 40 µm; which may thus consist of grains with an average size of less than 40, less than 39, less than 38, less than 37, less than 36, less than 35, less than 34, less than 33, less than 32, less than 31, less than 30, less than 29, less than 28, less than 27, less than 26 or less than 25 µm.

According to a particular embodiment, the cement used in the formulation according to the invention is in powder form, more particularly in the form of grains with an average size ranging from 1 µm to 40 µm.

According to a particular embodiment, the cement used in the formulation according to the invention is in powder form, more particularly in the form of grains with an average size ranging from 20 µm to 40 µm.

According to a particular embodiment, the cement used in the formulation according to the invention is in powder form, more particularly in the form of grains with an average size ranging from 1 µm to 10 µm.

According to a particular embodiment, the cement used in the formulation according to present invention is a phosphocalcic cement in powder form, more particularly in the form of grains with an average size of less than 40 µm, and even more particularly less than 20 µm.

According to a particular embodiment, the cement used in the formulation according to present invention is a magnesium phosphate cement in powder form, more particularly in the form of grains with an average size of less than 40 µm.

According to a particular embodiment, the formulation comprises from 40 to 70% w/w of cement in powder form, compared to the total weight of the formulation ; which may thus include 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 and 70% w/w of powder, compared to the total weight of the formulation.

According to a particular embodiment, the formulation is characterized in that:
- it comprises from 40 to 70% w/w of cement in powder form, compared to the total weight of the formulation ;
it comprises from 1 to 30% w/w of silylated polysaccharide, compared to the total weight of the formulation, said polysaccharide(s) being preferably selected from silylated hyaluronic acid, silylated chitosan, and mixtures thereof.

General methods for preparing cements, including phosphocalcic cements and magnesium phosphate cements are known in the Art. One example is detailed in WO2021/209616A1**.**

The cement used in the formulation according to the invention can thus be prepared by bringing into contact (e.g., by mixing) at least two phases, a solid phase and a liquid phase. When brought into contact, the at least two phases form a paste which progressively, and locally, sets and hardens into a solid mass, along with the dehydration of the liquid phase. Without wishing to be bound by the theory, it is proposed that those two phenomena contribute to the elastic properties of the cement following hydratatation and re-hydration steps.

The solid phase comprises one or several compounds selected from calcium phosphate (CaP) and magnesium phosphate compounds.

The liquid phase comprises polysaccharides, and more particularly silylated non-reticulated polysaccharides, such as silylated hyaluronic acid and silylated chitosan, to allow the dissolution of the initial compounds until the over-saturation of the solution, thus inducing the reprecipitation of crystals. The hardening of the cement takes place through the entanglement of needle-like or plate-like crystals.

For example, a calcium phosphate cement can be prepared by mixing 2 CaHPO₄ and CaCO₃ with a three-dimensional mixer, submitting the obtained product to isostatic compression (for example at 120 Mpa), further to a calcination step (for example at 1360°C during 15 hours), a compressed air quenching, grinding stages (for example submitting the mixture to a centrifugal crusher followed by a mortar crusher) and a final sieving step.

Size sieves can be for example of 40 µm and/or 20 µm.

According to a particular embodiment, the formulation according to the present invention, comprising the phosphocalcic cements and/or magnesium phosphate cements may thus be prepared by a method comprising a step of bringing into contact at least one solid phase and at least one liquid phase;
- said solid phase being characterized in that it comprises a calcium phosphate compound or a mixture of calcium and phosphate compounds or a magnesium phosphate compound or a mixture of magnesium and phosphate compounds;
- said liquid phase being characterized in that it comprises at least one silylated non-reticulated polysaccharide selected from silylated hyaluronic acid and silylated chitosan.

According to one embodiment, the hydrogel is formed after the solid and the liquid phase are brought into contact.

According to one embodiment, the hydrogel is formed while the solid and the liquid phase are brought into contact.

According to a particular embodiment, the liquid phase is characterized in that its pH is a basic pH; for example a pH at or above 10, for example a pH at or above 13.

According to particular non-mutually exclusive embodiment, the formulation according to the present invention, comprising the phosphocalcic cements and/or magnesium phosphate cements may be prepared by a method comprising a step of bringing into contact at least one solid phase and at least one liquid phase, in a [solid phase / liquid phase] mass ratio ranging from about 0.5 to about 2; in particular from about 0.9 to about 1.5.

Advantageously, the liquid phase may comprise a plurality of distinct silylated polysaccharides, in particular a plurality of distinct silylated non-reticulated polysaccharides, including at least one selected from silylated hyaluronic acid and silylated chitosan, and even more particularly a mixture of silylated hyaluronic acid and silylated chitosan.

Advantageously, the liquid phase may comprise one or more silylated polysaccharide(s), and one or more additional non-reticulated polysaccharide(s), the non-reticulated polysaccharide being optionally a functionalized polysaccharide or a non-functionalized polysaccharide.

Examples of non-reticulated polysaccharides, which are suitable according to the invention, may include hydroxypropylmethylcellulose (HPMC), hydroxyethylcellulose (HEC), carboxymethylcellulose (CMC), or a functionalized form thereof, native hyaluronic acid, starch, and poloxamers (e.g., pluronic F127).

Advantageously, the liquid phase may comprise or consist of a mixture of one or more silylated non-reticulated polysaccharide(s), and one or more reticulated polysaccharide(s); for example a mixture of (i) one or more silylated non-reticulated polysaccharide(s), such as silylated non-reticulated hyaluronic acid and/or silylated non-reticulated chitosan, and (ii) one or more silylated reticulated polysaccharide(s).

According to some embodiments, the liquid phase may comprise or consist of a mixture of (i) one or more silylated non-reticulated polysaccharide(s), and (ii) one or more non-silylated polysaccharide(s).

According to some embodiments, the liquid phase may comprise or consist of a mixture of (i) one or more silylated non-reticulated polysaccharide(s), (ii) one or more silylated reticulated polysaccharide(s), and (iii) one or more non-silylated polysaccharides.

Advantageously, the amount of the one or more silylated polysaccharide(s) should be present in mass excess, for example a two-fold excess or a three-fold excess, compared to the amount of the one or more non-reticulated polysaccharide(s).

Advantageously, the liquid phase may thus comprise:
- at least one non-reticulated polysaccharide which is hydroxypropylmethylcellulose (HPMC) or a functionalized form thereof;
- at least one silylated polysaccharide selected from silylated hyaluronic acid and silylated chitosan, and mixtures thereof.

Advantageously, the liquid phase may be a viscous solution comprising the silylated polysaccharides(s).

A formulation according to the present disclosure may thus be prepared by bringing into contact: **(i)** a solid phase comprising the one or more compounds selected from calcium phosphate (CaP) and magnesium phosphate compounds (e.g., α-TCP or Mg₃(PO₄)₂), and **(ii)** a liquid phase comprising silylated non-reticulated poysaccharides (e.g., silylated hyaluronic acid and/or silylated chitosan), or alternatively a precursor of said hydrogel comprising the silylated polysaccharides.

Advantageously, the silylated polysaccharides are present, in the formulation, in the form of a hydrogel of polysaccharides, and in particular in the form of a covalent hydrogel of polysaccharides.

In one particular embodiment, the hydrogel is a covalent hydrogel comprising at least 1 % in weight of silylated hyaluronic acid and/or silylated chitosan compared to the total weight of the formulation, in particular at least 2%, for example from 2% to 8% in weight, for example from 2% to 4% in weight of silylated hyaluronic acid and/or silylated chitosan compared to the total weight of the formulation.

In particular, the hydrogel is a covalent hydrogel comprising silylated, and reticulated, hyaluronic acid.

Still particularly, the covalent hydrogel comprises silylated hyaluronic acid with a molecular weight ranging from 50 kDa to 3 Mda, for example ranging from 120 kDa to 3 Mda, for example ranging from 420 kDa to 2.88 Mda.

A covalent hydrogel as used in the formulation according to the invention can be prepared by dissolving the lyophilized silylated polysaccharide, for example silylated hyaluronic acid, in a aqueous solution, for example of pH at or above 13, or by dissolving the powder of silylated polysaccharide, for example silylated chitosan, in an aqueous acidic solution, for example of pH ranging from 1 to 3.

A covalent hydrogel as used in the formulation according to the invention can be prepared for example according to the method described in WO2011089267**.**

According to a particular embodiment, the silylated polysaccharide (e.g., the silylated hyaluronic acid) can be prepared through amidation between the carboxylic acid of the polysaccharide and the primary amine of aminopropyltriethoxysilane (APTES) can be conducted. To facilitate this reaction, an activating agent, such as 4-(4,6-Dimethoxy-1 ,3,5-triazin-2-yl)-4-methylmorpholinium chloride (DMTMM), can be used.

In another embodiment, the method according to the invention also comprises a sterilization step, wherein in particular the cement and the silylated polysaccharides, or hydrogel thereof, of the formulation can be sterilized by different means and separately.

In another embodiment, the bone implant is sterilized after the 3D printing step and before being brought into contact with the medium.

Accordingly, in one embodiment, the bone implant is provided in a sterile form.

In one embodiment, the formulation is prepared in a sterile room or a biosafety cabinet.

According to a **further embodiment,** the invention relates to the use of a formulation comprising **(i)** a magnesium phosphate cement and **(ii)** an hydrogel of polysaccharides, for 3D printing bone implants; preferably characterized in that the polysaccharides comprise at least one selected from: silylated hyaluronic acid and silylated chitosan.

According to a **further embodiment,** the invention relates to a kit for 3D printing bone implants comprising:
(i) a magnesium phosphate cement; and
(ii) silylated non-reticulated polysaccharides, said silylated polysaccharides being preferably selected from: silylated hyaluronic acid and silylated chitosan polysaccharides.

According to a **further embodiment,** the invention relates to a method to prepare a formulation for 3D printing bone implants, comprising a step of bringing into contact (i) a magnesium phosphate cement and (ii) silylated non-reticulated polysaccharides ; preferably characterized in that the silylated polysaccharides comprise at least one selected from : silylated hyaluronic acid and/or silylated chitosan polysaccharides.

According to a **further embodiment,** the invention relates to a method to prepare a 3D bone implant, comprising at least the steps of :
a) providing a formulation comprising **(i)** a magnesium phosphate cement and **(ii)** silylated polysaccharides or a hydrogel thereof; preferably characterized in that the silylated polysaccharides comprise at least one selected from: silylated hyaluronic acid and silylated chitosan;
b) 3D printing said formulation, thereby producing the bone implant;
c) bringing the bone implant in contact with a medium, such as a biological medium and/or a medium with pH equal or superior to 4.

According to a **further embodiment,** the invention relates to bone implants prepared according to the above-mentioned methods.

In particular, the present disclosure further relates to a method for treating or preventing bone disorders or bone defects in a subject, comprising the steps of:
a) providing a 3D bone implant according to the present disclosure;
b) optionally drying the bone implant;
c) optionally bringing the bone implant in contact with a medium, such as a biological medium and/or a medium with pH equal or superior to 4;
d) optionally further modifying the bone implant, thereby producing a customized form of the bone implant;
e) inserting the bone implant in the subject.

Advantageously, the step of modifying the bone implant, thereby producing a customized form of the bone implant, can be performed entirely *in vitro* and/or by a physician.

Alternatively, the step of modifying the bone implant, thereby producing a customized form of the bone implant, can be performed *in vivo* in the context of a therapeutic method; in which case the corresponding method necessarily includes a step of inserting and/or administering the bone implant to the individual in need thereof.

### General definitions

The term "magnesium phosphate cement" means a cement wherein the pulverulent solid phase (or powder component) is made of a magnesium phosphate compound or a mixture of magnesium and phosphate compounds. In a non-exhaustive manner, the phosphate compound may comprise potassium dihydrogen phosphate, sodium dihydrogen phosphate, calcium dihydrogen phosphate or a mixture thereof.

The term "phosphocalcic cement (CPC)" or "calcium phosphate cement" means a cement wherein the pulverulent solid phase (or powder component) is made of a calcium phosphate compound or a mixture of calcium and phosphate compounds. Varying compositions of CPC are commercially available. In the context of the present invention, the term "phosphocalcic" or "calcium phosphate" refers to minerals containing calcium ions (Ca²⁺) together with orthophosphate (PO₄³⁻), metaphosphate or pyrophosphate (P₂O₇⁴⁻) and occasionally other ions such as hydroxide ions or protons. Phosphocalcic cements, according to the disclosure, may include those corresponding to apatitic cements (e.g., hydroxyapatite cements or calcium deficient hydroxyapatite cements) and those corresponding to brushitic (dicalcium phosphate dihydrate, DCPD) cements.

The term "apatite cement" or "apatitic cement" may refer, in particular, to cements made by α-TCP. The term "brushite cement" or "brushitic cement" may refer, in particular, to cements made by β-TCP.

The term "tricalcium phosphate (TCP)" refers to the compound of formula Ca₃(PO₄)₂ which is also known as calcium orthophosphate, tertiary calcium phosphate, tribasic calcium phosphate or bone ash. The term α-TCP refers, more specifically, to formula α-Ca₃(PO₄)₂. The term β-TCP refers, more specifically, to formula β-Ca₃(PO₄)₂.

The term "average size" (or "average grain size" or "mean particle size") denotes the mean equivalent diameter of said particles measured by LASER diffraction analysis.

The term "hydrogel" means a network of polymer chains that are water-insoluble, in which water is the dispersion medium. The term may thus refer to a water-insoluble network of reticulated compounds (e.g., reticulated polysaccharides).

The term "physical hydrogel" means a hydrogel that can undergo a reversible transition from liquid (solution) to a gel in response to a change in environmental conditions such as temperature, ionic concentration, pH, or other conditions such as mixing of two components. The network is formed through molecular entanglements and/or secondary forces including hydrogen bonding, hydrophobic forces and electrostatic interactions.

The term "covalent hydrogel" means covalent bonding that introduces mechanical integrity and degradation resistance compared to other weak material. Chemical crosslinking relies on the formation of covalent bonds between reacting groups grafted to the polymer backbone that will crosslink under specific conditions. Generally, carboxyl, hydroxyl and amine are the most targeted groups. Organo-mineral moieties like silanols can also be used.

The term "polysaccharide" means a polymer made up of many monosaccharides joined together by glycosidic bonds. Natural and synthetic polysaccharides are included. Examples of polysaccharides are cellulose and derivatives thereof, include hydroxypropylmethylcellulose (HPMC), hydroxyethylcellulose (HEC), carboxymethylcellulose (CMC), pectin, chitosan, hyaluronic acid, and mixtures thereof.

The term "hyaluronic acid" refers to the primary component of the extracellular matrix of human connective tissues. Hyaluronic acid is a high molecular weight linear polysaccharide of alternating D-glucuronic acid and N-acetyl-D-glucosamine that constitutes the backbone of the extracellular matrix (ECM).

The term "chitosan" refers to a linear polysaccharide composed of randomly distributed β-(1,4)-linked D-glucosamine (deacetylated unit) and N-acetyl-D-glucosamine (acetylated unit).

The term "silylated polysaccharide" means any organic or synthetic polysaccharide onto which are grafted an organo-mineral silyl function, preferably an alkoxysilane. Silylation allows the formation of covalent bonds between the polysaccharides constituting the hydrogel as a function of pH. The silylated biomolecules are thus able to form a pH-dependent self-reticulating hydrogel. Silylated polysaccharides may thus be either reticulated polysaccharides or non-reticulated polysaccharides. When silylated polysaccharides are part of an hydrogel, they may thus be reticulated (e.g., partially reticulated or completely reticulated) silylated polysaccharides.

In the sense of the present invention, the term "hydrogel of polysaccharides" refers more particularly to a hydrogel comprising reticulated silylated polysaccharides, in particular those selected at least one from: silylated reticulated hyaluronic acid and silylated reticulated chitosan.

The term "precursor hydrogel" means a viscous solution of macromolecules before reticulation (e.g., crosslinking) and thus before becoming a gel (i.e. an insoluble solid).

The term "printing" refers to the deposition of a material, here the formulation according to the disclosure, using a print head, nozzle, or any other printer technology.

The term "3D printing" refers to any technology used to fabricate physical objects from computer generated, e.g., computer-aided design (CAD), data sources. It is the fabrication of objects (e.g., bone implants) through the deposition of a material (e.g., a formulation according to the present disclosure) using a print head, nozzle, or any other printer technology.

The term "3D printer" refers to any device/machine suitable for "3D printing", especially the 3D printing of bone implants. Hence, the term "3D-printed formulation or implant" refers to any formulation or implant obtained by 3D printing.

The term "bone implant", or "bone graft" is meant to refer to any type of bone implant and structure thereof, which may be relevant to tissue engineering application, which may thus include meniscal, cartilage, and subchondral bone replacement and regeneration (e.g., for osteoarthritis, cartilage defects, and damaged meniscal tissue); other cartilaginous tissues (e.g., ear, nose, esophagus, trachea); ligament-bone fixation devices for improving integration and restoring mechanical function after ligament repair surgery; craniofacial regenerative implants (e.g., skull plate, nose, cheek bone); support and regeneration of tissue following corrective surgeries treating cleft pallet; alveolar ridge support and regeneration immediately following or long after tooth removal; spine fusion and regeneration; regeneration in any long bones, hip bones, or bones in the extremities (e.g., hand, wrist, ankle, foot, toes); drug, gene, or growth factor delivery; and biodegradable implants or coatings.

The term "bone disorder" or "bone defect" may thus refer to, in a non-exhaustive manner, any Disorder related to bone, cartilage, tendon or tooth defects, such as any known disorder wherein bone, cartilage, tendon or periodontal healing or reconstruction, e.g., regeneration, is desired. Non-limiting examples of treatments of disorders related to bone, cartilage, tendon or periodontal defects or diseases or the like are regeneration, repair and growth of bone and periodontal tissue; regeneration, repair and growth of bone in mammals, such as human or any other animal; treatment of abnormalities of bone formation or regeneration; wound healing, ectopic bone induction and healing of segmental bone defects in vertebrates; treatment of skeletal disorders and deformations; repair of large bone defects originating from trauma, excision of tumors or congenital malformations, reconstructing bone stocks worn off by an implanted endoprosthesis in revision operations and healing delayed or non-united fractures; repair of bone and cartilage defects such as critical size defects, non-critical size defects, non-union fractures, segmental non-union of fractures; acute fractures, chondral defects, osteochondral defects, subchondral defects; local bone and cartilage formation; defects resulting from degenerative diseases; dental applications such as repair of periodontal tissues, alveolar bone, cementum, tooth root membrane, filling of the tooth root canal and improvement or enhancement of fixation of the dental implant.

As used herein, the term *"biological material"* means any material comprising biological cell material, such as living or dead cells, preferably living cells, including autologous and allogenic living cells, as well as any secreted factors derived from the culturing of such cells.

In certain preferred embodiments, however, the present invention pertains to cell material comprising living biological cells. The expression "biological cell" in context of the invention shall preferably refer to a mammalian cell, most preferably a human cell. Under certains embodiments of the invention, a biological cell may thus encompass tissue-forming cells and/or undifferentiated or dedifferentiated cells, including osteoblasts, chondrocytes, fibroblasts, endothelial cells, and myocytes. Certain embodiments of the invention pertain to mesenchymal stromal cells as preferred biological cells in context of the invention. This may in some embodiments include other cells that can be derived from bone marrow fractions, such as for example hematopoietic progenitor cells, or erythropoietic progenitor cells. In the present disclosure, the term "mesenchymal stromal cell" is a multipotent stromal cell that can differentiate into a variety of cell types, including, but not limited to osteoblasts (bone cells), chondrocytes (cartilage cells), fibroblasts, endothelial cells, myocytes (muscle cells) and adipocytes (fat cells). These cells are also known as *"mesenchymal stem cells"* due to their multipotency.

As used herein, the term *"biological material"* may thus also comprise or consist of any material comprising growth factors, especially bone growth factors or cytokines. Such growth factors and cytokines are often added in order to render cells in bone graft material osteoinductive. However, the materials of the present invention in all aspects and embodiments thereof preferably do not require such addition of osteo-inductive cytokines or growth factors since the cellular compositions of the invention are inherently osteo-inductive. Such cytokines include bone morphogenic protein (BMP), Indian hedgehog (IHH), transforming growth factor b (TGF3); fibroblast growth factors (FGFs); Wnt ligands and b-catenin; insulin-growth factors (IGFs); collagen, such as type I collagen; Runx2; osteopontin; osterix; vascular endothelial growth factor (VEGF); platelet derived growth factor (PDGF); osteoprotegerin (OPG); NELlike protein 1 (NELL-I); or a mixture thereof.

As used herein, *"treating"* means any manner in which one or more of the symptoms of a bone defect, bone disease or bone disorder are ameliorated or otherwise beneficially altered. As used herein, amelioration of the symptoms of a particular disorder refers to any lessening of the symptoms, whether permanent or temporary, lasting or transient, that can be attributed to or associated with treatment by the compositions and methods of the present invention. Accordingly, the expression *"treating"* may include *"reversing partially or totally the effect"* of a given condition, or even *"curing"* when permanent reversal is considered.

As used herein, *"preventing"* encompasses *"reducing the likelihood of occurrence"* and *"reducing the likelihood of re-occurrence"* and *"delaying the likelihood of occurrence or reoccurrence".*

As used herein, the term *"subject"* or *"patient"* may encompass an animal, human or non-human, rodent or non-rodent. Veterinary and non-veterinary applications are contemplated. The term includes, but is not limited to, mammals, e.g., humans, other primates, pigs, rodents such as mice and rats, rabbits, guinea pigs, hamsters, cows, horses, cats, dogs, sheep and goats. Typical subjects include humans, farm animals, and domestic pets such as cats and dogs.

As used herein, the singular form *"a", "an"* and *"the"* include plural references unless the context clearly dictates otherwise. For example, the term *"a pharmaceutically acceptable carrier"* encompasses a plurality of pharmaceutically acceptable carriers, including mixtures thereof.

As used herein, « *a plurality of »* may thus include « *two* » or « *two or more ».*

As used herein, « *comprising»* may include « *consisting of ».*

As used herein, the term *"biocompatible"* is meant to refer to compounds (e.g., nanoparticles) which do not cause a significant adverse reaction in a living animal when used in pharmaceutically relevant amounts.

As used herein, a *"pharmaceutically acceptable carrier"* is intended to include any and all carrier (such as any solvents, dispersion media, coatings, antibacterial and antifungal agents, isotonic and absorption delaying agents, and the like) which is compatible with pharmaceutical administration. The use of such media and agents for pharmaceutically active substances are known. Except insofar as any conventional media or agent is incompatible with the active compound, such media can be used in the compositions of the invention.

### EXAMPLES

### Material & Methods

In order to prepare hybrid CPC cements and hybrid MPC cements according to the invention, the proportions of each phase (solid vs. liquid) have been preliminarily adjusted in order to obtain a printable ink with a 25 cone (internal diameter of 250 µm) and a printing pressure inferior to 700 kPa. Unless stated otherwise, the proportions are expressed as mass percentages, compared to the total weight of the formulation (e.g., CPC formulation or CPM formulation).

**Table 1: proportion of each phase (solid vs. liquid) in the tested cements.**

| | Solid phase (%) | Liquid phase (%) | Solid / liquid Ratio |
|---|---|---|---|
| CPC | 60 | 40 | 1.5 |
| MPC | 47.5 | 52.5 | 0.9 |

The provided experiments are meant to illustrate the mechanical properties of the printable formulations according to the invention, in order to satisfy the following requirements: **(i)** being suitable for storage and printing at ambient temperature, **(ii)** being flexible after hydration in a buffer solution, and then **(iii)** being able to rigidify *in situ* in a biological environment.

### Liquid phase

The liquid phase is an aqueous phase which may further contain ions, such as those introduced by a phosphate buffer solution. Advantageously, it can also be supplemented with macromolecules which may reticulate in order to form an hydrogel of polysaccharides.

The selection of a particular set of polysaccharides provides novel properties to the ink, both with respect to its injectability and to the mechanical properties of the obtained 3D implant.

For control samples, the liquid phase does not contain any macromolecules and only consists of a NaH₂PO₄ solution. It is thus an ionic solution, in the sense that upon mixing with the precursor, the Na+ and H₂PO₄- ions will fasten the hardening of the material.

The liquid phase reported hereafter as the *"viscous"* phase consists of a mixture of a NaOH solution at a concentration of 0.1 mol/L in which two polysaccharides are dissolved, namely polysaccharide 1 (P1), which is a viscosity agent, in particular HPMC (e.g., HPMC Methocel^{™}), and polysaccharide 2 (P2), which is a functionalized polysaccharide allowing the formation of a specific hydrogel.

With respect to the present invention, the polysaccharide 2 (P2) can be selected from silylated non-reticulated hyaluronic acid and silylated non-reticulated chitosan; in particular silylated hyaluronic acid (siHA)..

In order to improve the printability of the obtained ink, the composition of the hydrogel will vary depending on the type of cement (i.e. phosphocalcic cement or magnesium phosphate cement), according to the following table:

**Table 2: composition of the liquid and solid phases for obtaining hybrid CPCs and MPCs according to the invention.**

| | **Solid Phase** | **Liquid phase** | |
|---|---|---|---|
| | | Viscous phase | Ionic phase |
| CPC | 100 % αTCP | 1.75% P1 (e.g., HPMC) | 100% NaH₂PO₄ |
| | | 6.5% P2 (e.g., siHA) | |
| MPC | 100% Mg₃(PO₄)₂ | 2.5% P1 (e.g., HPMC) | 100% NaH₂PO₄ |
| | | 5.5 % P2 (e.g., siHA) | |

Unless stated otherwise, the above-mentioned percentages are expressed as mass percentages, compared to the total weight of the corresponding phase (e.g., viscous phase ).

In order to prepare the viscous phase, P1 and P2 are sequentially dissolved in in a syringe and incubated for two hours prior to the addition of each polymer. Then, the composition is homogeneized with a connector between two syringes, and the resulting formulation is then centrifugated at 2000 rpm for five minutes and kept for 24h.

### Solid phase

Phosphocalcic cements (CPCs) prepared according to the following protocol include α-TCP (α tricalcic phosphate). In order to obtain α-TCP suitable for the preparation of the CPC formulation, the starting material is CDHA powder (or Calcium-deficient hydroxyapatite), heaten up at high temperature. At 800°C, the original compound shifts toward a cristalline phase. At around 800°C, the original compound shifts to a β-TCP form, and then at around 1200°C shifts to a α-TCP form. One method for preparing α-TCP is disclosed in WO2021/209616**.**

Magnesium phosphate cements (MPCs) prepared according to the following protocol are characterized by a starting material consisting of thin-grained (between 1 and 10 µm) hydrated magnesium phosphate.

The two phases are then mixed in a mortar until an homogeneous paste is obtained in varying proportions of solid vs. liquid phase. The paste is then injected in a mold, and stored for 24h to 48h for future three-point flexural tests. The analysis is performed on dry and rehydrated materials.

The hydration step may be performed in a NaH₂PO₄ solution at 0.1 mol/L or in a phosphate saline buffer (PBS) solution. This last condition is meant to reproduce *in vivo* conditions. Advantageously, the proposed protocol is further applicable to biological fluids, such as bone and bone-derived samples.

### Example 1. Hybrid phosphocalcic cements.

Hybrid CPCs (i.e. CPCs associated with hydrogels according to the invention) differ from control CPCs in that their mechanical properties can be modulated after hydration.

When such hybrid CPCs are hydrated, their ε strain constant is about five times more important than for dry CPCs. Advantageously, the Young modulus remains low and never exceeds 3 MPa whereas the σ stress is generally below 0.5 MPa in the tested conditions.

For hybrid CPCs, the length of the hydration time does not appear to have a signicant impact upon the mechanical properties. Overall, the behavioural change appears to be mostly linear, although a slight decrease of the Young modulus and of the ε strain constraint is observed.

**Table 3: mechanical properties of hybrid phosphocalcic cements (CPCs) according to the invention, as a three-point flexural test. N refers to the number of trials. E refers to Young modulus. The σ constant refers to the stress constraint. The ε refers to the strain induced deformation.**

| **Sample** | **N** | **E (MPa)** | **σ stress (MPa)** | **ε strain (%)** |
|---|---|---|---|---|
| Dry control | 4 | 230.4 +/- 154.6 | 3.2 +/- 0.4 | 2.5 +/- 0.6 |
| Hardened control | 4 | 217.9 +/- 94.0 | 5.7 +/- 1.6 | 4.1 +/- 0.6 |
| Dry hybrid | 4 | 85.8 +/- 28.6 | 2.6 +/- 0.3 | 4.7 +/- 1.1 |
| Hydrated hybrid after 20 minutes | 3 | 2.8 +/- 0.4 | 0.5 +/- 0.1 | 23.6 +/- 2.7 |
| Hydrated hybrid after 40 minutes | 3 | 1.9 +/- 0.2 | 0.3 +/- 0.1 | 20.2+/-1.1 |
| Hydrated hybrid after 70 minutes | 3 | 1.5 +/- 0.2 | 0.3 +/- 0.0 | 21.7 +/- 1.7 |
| Hydrated hybrid after 20 minutes (**+ 48h PBS**) | 3 | 15.0 +/- 4.9 | 1.6 +/- 0.4 | 12.7 +/- 1.8 |
| Hydrated hybrid after 40 minutes (**+ 48h PBS**) | 3 | 24.3 +/- 5.1 | 1.9 +/- 0.2 | 10.7 +/- 1.1 |
| Hydrated hybrid after 70 minutes (**+ 48h PBS**) at 37°C | 3 | 29.3 +/- 17.2 | 2.0 +/- 0.2 | 10.5 +/- 4.1 |

The hardening of the hybrid cement can be detected through the decrease of the flexibility of the material and an increase of its rigidity.

It thus observed that deformability has then decreased of about its half (ε = 10%) when compared to samples which only have been hydrated, but remains constant for all the tested lengths of hydration times (20, 40 or 70 minutes).

It is also superior to dry hybrid CPC and even more when compared to dry control CPCs outside of the invention.

In view of the above, it is shown herein that the presence of an hydrogel in the hybrid CPCs of the invention leads to a deformable material which is particularly convenient for obtaining personalized 3D bone implants.

### Example 2. Hybrid magnesium phosphate cements.

Compared to phosphocalcic cements (CPCs), magnesium phosphate cements (MPCs) appear to exhibit superior Young modulus and stress constants (E > 400 Mpa and 4.7 Mpa < σ < 13.4 Mpa) but the observed strain is slightly lower ( 1.7% < ε < 4.7 %).

After hydration, the mechanical properties of hybrid MPCs according to the invention tend to be modified significantly over time, when compared to control MPCs.

Interestingly, it is shown herein that this modification is neither linear nor constant, with the highest ε strain constant after 40 minutes of hydration.

**Table 4: mechanical properties of hybrid magnesium phosphate cements (MPCs) according to the invention as a three-point flexural test. N refers to the number of trials. E refers to Young modulus. The σ constant refers to the stress constraint. The ε refers to the strain induced deformation.**

| **Sample** | **N** | **E (MPa)** | **σ stress (MPa)** | **ε strain (%)** |
|---|---|---|---|---|
| Dry control | 4 | 448,0 +/- 103.8 | 4.7 +/- 0.8 | 1.7 +/- 1.0 |
| Hardened control | 4 | 402.1 +/- 97.8 | 13.4 +/- 3.4 | 4.7 +/- 1.5 |
| Dry hybrid | 7 | 333.8 +/- 152.6 | 8.3 +/- 1.3 | 3.3 +/- 1.3 |
| Hydrated hybrid after 20 minutes | 3 | 82.1 +/- 7.7 | 5.2 +/- 0.3 | 5.9 +/- 0.2 |
| Hydrated hybrid after 40 minutes | 3 | 10.3 +/- 0.3 | 2.0 +/- 0.4 | 17.2 +/- 3.7 |
| Hydrated hybrid after 70 minutes | 3 | 39.5 +/- 3.2 | 2.9 +/- 0.1 | 8.5 +/- 0.7 |
| Hydrated hybrid after 20 minutes (**+ 48h PBS**) | 3 | 6.5 +/- 1.6 | 0.5 +/- 0.1 | 9.6 +/- 0.8 |
| Hydrated hybrid after 40 minutes (**+ 48h PBS**) | 3 | 8.7 +/- 5.8 | 0.8 +/- 0.2 | 14.3 +/- 7.8 |
| Hydrated hybrid after 70 minutes (**+ 48h PBS**) at 37°C. | 6 | 44.4 +/- 29.8 | 1.8 +/- 0.3 | 5.8 +/- 2.4 |

In view of the above, it is shown herein that the presence of an hydrogel in the hybrid MPCs of the invention leads to a deformable material which is particularly convenient for obtaining personalized 3D bone implants.

### Example 3. Treatment of cleft palate deformity in a non-human mammal (dog).

A 3D personalized bone implant is used herein to treat six dog patients displaying a spontaneous cleft palate deformity; either unilateral or bilateral).

The 3D bone implants are obtained from a combination of a phosphocalcic cement (CPC) and an hydrogel of polysaccharides according to the present disclosure.

A few weeks before surgery, live CT scan is performed for imaging hard tissue defects. A virtual 3D model is then designed with the associated personalized implant.

The 3D bone implant is printed with a honeycomb internal structure (20% density) using BioX (Cellink) or RGen200 (RegenHU) printer using a 25G or 27G cone. Calcium-phosphate based organo-mineral paste described in the previous examples was used to generate the 3D personalized implant. Implants were produced several days before surgery.

Surgery was carried out under approval of the regulatory authorities.

The implant is disinfected then rehydrated in a saline buffer for 30 min. The excess of saline buffer is removed using sterile gauze, then the implant is soaked in autologous bone marrow freshly harvested for at least 30 min.

Soft tissue reconstruction is carried out to close the communication between the oral and nasal cavity by carefully dissecting the oral and nasal mucosa and selectively stitching them. A pocket is created to place the bone marrow loaded implant. Then the pocket is closed and soft tissue reconstruction is finalized (lip and palate).

Three months after surgery, CT scan is performed, which demonstrates scaffold osteointegrated biodegradation ongoing, along with new bone formation within the implant macroporosity.

## Claims

1. Use of a formulation comprising **(i)** a calcium phosphate cement or a magnesium phosphate cement and **(ii)** an hydrogel of polysaccharides, for 3D printing bone implants; **characterized in that** the polysaccharides comprise at least one selected from: silylated hyaluronic acid and silylated chitosan.

2. Use according to claim 1, wherein said hydrogel is a hydrogel of silylated hyaluronic acid.

3. Use according to claim 2, wherein said hydrogel is a hydrogel of polysacharides further comprising hydroxypropylmethylcellulose (HPMC) or a silylated form thereof.

4. Use according to any of claims 1 to 3, wherein said cement is an apatitic calcium phosphate cement.

5. Use according to any of claims 1 to 4, wherein the ratio hydrogel/cement is of 2:3 w/w.

6. Use according to any of claims 1 to 5, wherein said hydrogel comprises from 2 to 4% w/v of silylated hyaluronic acid and/or silylated chitosan, of the total volume of the hydrogel.

7. A kit for 3D printing bone implants comprising:
(i) a calcium phosphate cement and/or a magnesium phosphate cement; and
(ii) silylated non-reticulated polysaccharides, said polysaccharides comprising at least one selected from: silylated hyaluronic acid and silylated chitosan polysaccharides.

8. A method to prepare a formulation for 3D printing bone implants, comprising a step of bringing into contact (i) a calcium phosphate cement or a magnesium phosphate cement and (ii) silylated non-reticulated polysaccharides comprising at least one selected from : silylated hyaluronic acid and/or silylated chitosan polysaccharides.

9. A method to prepare a 3D bone implant, comprising at least the steps of :
a) providing a formulation comprising **(i)** a calcium phosphate cement or a magnesium phosphate cement and **(ii)** an hydrogel of polysaccharides comprising at least one selected from: silylated hyaluronic acid and silylated chitosan;
b) 3D printing said formulation, thereby producing the bone implant, and optionally drying the bone implant;
c) bringing the formulation in contact with a medium, such as a biological medium and/or a medium with pH equal or superior to 4.

10. A method to prepare a 3D bone implant, comprising at least the steps of :
a) providing a formulation comprising **(i)** a phosphocalcic cement and **(ii)** an hydrogel of polysaccharides;
b) 3D printing said formulation, thereby producing the bone implant, and optionally drying the bone implant;
c) bringing the formulation in contact with a medium, such as a biological medium and/or a medium with pH equal or superior to 4;
d) further modifying the bone implant after step d), thereby producing a customized form of the bone implant.

11. The method according to claim 8 or 9 or 10, which further comprises a step of mixing the cement and/or the polysaccharides with a biological material prior to 3D printing.

12. Use of a formulation comprising **(i)** a magnesium phosphate cement and **(ii)** an hydrogel of polysaccharides, for 3D printing bone implants; preferably **characterized in that** the polysaccharides comprise at least one selected from: silylated hyaluronic acid and silylated chitosan.

13. A kit for 3D printing bone implants comprising:
(i) a magnesium phosphate cement; and
(ii) silylated non-reticulated polysaccharides, said polysaccharides comprising preferably at least one selected from: silylated hyaluronic acid and silylated chitosan polysaccharides.

14. An implant as prepared according to any of claims 9, 10 or 11 or 12.

15. Implant according to claim 14, further comprising growth factors and/or cells.
